# EUROPEAN PATENT SPECIFICATION

(11) **EP 1 024 136 B1**
(45) Date of publication and mention of the grant of the patent: **14.03.2007**
(21) Application number: 00300616.0
(22) Date of filing: 27.01.2000
(51) Int. Cl.: C07C 315/02, C07C 317/04

(54) **A process for producing dimethyl sulphoxide**
Verfahren zur Herstellung von Dimethylsulfoxid
Procédé pour la préparation de diméthylsulfoxyde

(30) Priority: 29.01.1999 JP 2156199
(43) Date of publication of application: 02.08.2000
(73) Proprietor: Toray Fine Chemcials Co. Ltd., Moriyama-shi, Shiga 524-0041 (JP)
(72) Inventor: Fukui, Yoshiyuki, Takashima-gun, Shiga 520-1121 (JP); Aburai, Kouji, Otsu-shi, Shiga 520-0842 (JP); Sakamoto, Kosuke, Odakaryokuchi Koen 701, Nagoya-shi, Aich 458-0832 (JP)
(74) Representative: Coleiro, Raymond

(56) References cited:
- US-A- 2 702 824
- US-A- 2 935 532

## Description

The present invention relates to a process for producing dimethyl sulfoxide (DMSO) industrially widely used, for example, as a reaction solvent of medical and agricultural intermediate products, a synthetic reagent or as a special detergent for electronic material. In more detail, the present invention relates to an improvement in the process of continuously oxidizing dimethyl sulfide (DMS) using NOx (which may be any one or more, and preferably a mixture, of NO, N₂O₃, NO₂ and N₂O₄) as a catalyst in a liquid phase for producing DMSO.

US 2,935,532 discloses a process for production of dialkyl sulfoxides which comprises the liquid phase oxidation of a stoichiometric excess of a dialkyl sulfide with a solution of nitrogen dioxide in a dialkyl sulfoxide.

US-A-2,702,824 describes a process in which gaseous NOₓ is passed through liquid dimethylsulfoxide and thereafter recycled as a gas into the process.

As a conventional process for producing DMSO, a process of continuously oxidizing DMS using NOx as a catalyst in a liquid phase is publicly known (US-A-2825744 and JP-B-42-009771).

In the DMS oxidizing reaction, NOx gas to be used as a catalyst is supplied together with a gas mainly composed of oxygen to be used as an oxidizing agent to a DMS-containing liquid phase reaction system, and is released outside the reaction system as a reaction off gas, to be treated by an alkali for dumping. The amount of NOx gas used is as much as required as a catalyst for DMS. In the oxidation reaction, new NOx is kept supplied as a catalyst to the reaction system, and the used catalyst NOx is dumped. Thus, since the conventional process requires a considerable amount of catalyst NOx, the dumping of used catalyst NOx is undesirable in view of cost and effective utilization of a resource.

Furthermore, the amount of oxygen gas used as an oxidizing agent in this process is generally an approximately theoretical amount for liquid phase DMS or slightly larger than it. However, the highly pure oxygen gas with an oxygen concentration of 99% or more used here is high in purchase price, and requires large equipment such as a receiving tank for use of oxygen.

The present invention addresses the problem of providing an efficient DMSO production process improved in the conversion from DMS to DMSO in the continuous oxidation reaction of DMS for producing DMSO by recycling NOx used as a catalyst, in order to provide an improvement over the disadvantageous practice of dumping the used catalyst, as in the known processes, more specifically, to provide a DMSO production process improved in the recovery rate and/or absorption rate of NOx by recovering NOx from the reaction off gas and/or the gas removed from the reaction product solution, in the reaction to continuously oxidize DMS by a gas mainly composed of oxygen, using NOx as a catalyst in a liquid phase for producing DMSO.

The present invention also addresses the problem of providing a low-cost DMSO production process allowing the use of simpler production equipment, by using relatively low purity oxygen gas obtained by the pressure swing adsorption treatment of air.

We made intensive studies in an attempt to solve the above problems. As a result, we found that dimethyl sulfoxide can be produced very efficiently at a high yield, by effectively supplying at least partially the reaction product solution containing dimethyl sulfoxide (DMSO) and NOx obtained by oxidation reaction of dimethyl sulfide (DMS) to the oxidation reaction for recycling. In embodiments of the invention, the respective DMSO and NOx components of the reaction product to be recycled may become separated from one another and then recombined to form a solution for recycling.

Accordingly, the present invention provides a process for producing dimethyl sulfoxide, in which dimethyl sulfide is continuously oxidized by a gas mainly composed of oxygen using NOx as a catalyst in a liquid phase, comprising the step of supplying at least partially the dimethyl sulfoxide and NOx of the reaction product solution obtained by said oxidation reaction, as a reaction solution to the oxidation reaction for recycling.

According to embodiments of the present invention, there is provided a process for producing dimethyl sulfoxide, in which dimethyl sulfide is continuously oxidized by a gas mainly composed of oxygen using NOx as a catalyst in a liquid phase using an oxidation reactor, comprising the steps of allowing the dimethyl sulfoxide solution to absorb NOx present in reaction off gas in an absorbing column, and supplying the NOx-containing DMSO solution to the oxidation reactor for recycling. It is preferable to employ NOx both from the reaction product solution and from the off-gas in a DMSO solution as the recycled solution. It is also preferable to use the oxygen gas obtained by the pressure swing adsorption treatment of air described later, as the gas mainly composed of oxygen.

A gas mainly composed of oxygen contains no gas other than oxygen present in an amount by weight greater than oxygen and preferably contains at least 50%, more preferably at least 75%, especially at least 80% of oxygen by weight of the total weight of the gas.

Embodiments of the DMSO production process of the present invention also includes the following preferred steps.
(1) Nitrogen gas is blown into the reaction product solution at a temperature higher than the reaction temperature, for bringing nitrogen into contact with the reaction product solution, to remove NOx from the reaction product solution mainly composed of NOx and DMSO.
(2)Reaction off gas and/or gas mainly composed of NOx removed from the reaction product solution is sent to a NOx absorbing column, for recovering NOx.
(3) The reaction product solution remaining after removing NOx is used as the NOx absorbable solution.
(4) NO in the reaction off gas and/or in the gas removed from the reaction product solution is oxidized into NO₂ which is then sent to a NOx absorbing column, for recovering NOx.
(5) A gas with an oxygen concentration of 80 to 96% obtained by pressure swing adsorption treatment is used.

Embodiments of the present invention are now described in more detail.

The present invention addresses the problem of providing an improvement of the process for continuously oxidizing DMS by a gas mainly composed of oxygen using NOx as a catalyst in a liquid phase for producing DMSO. In a process embodying the present invention, there are several preferable conditions as described below for improving the conversion from DMS to DMSO in an oxidation reaction, and for improving the recovery rate and/or absorption rate of NOx used as a catalyst, and also for allowing the use of a low purity oxygen gas as an oxidizing agent.

The oxidation reaction of DMS in the present invention is exothermic. So, although various types of oxidation reactors are available for the oxidation reaction of DMS, a perfectly mixing reactor and a multitubular reactor are preferably used. A perfectly mixing reactor is especially preferably used.

In a process embodying the present invention, it is preferable that the temperature in the oxidation reactor is 10°C to 50°C. A more preferable range is 20°C to 40°C. If the temperature is lower than 10°C, the produced DMSO may be coagulated, not allowing the reaction to be continued. If higher than 50°C, dimethyl sulfone (hereinafter called DMSO₂) may be produced, by further oxidizing DMSO, as a byproduct, possibly in a large amount.

It is preferable that the pressure in the reactor during the oxidation reaction is atmospheric pressure, but the reaction can take place even at a higher pressure.

It is preferable that the catalyst NOx concentration used in the DMS oxidizing reaction in the present invention is 0.01 to 0.2 as NOx/DMS ratio by weight. A more preferable range is 0.05 to 0.12 as the NOx/DMS ratio by weight. If the reaction is effected at a NOx/DMS ratio by weight of lower than 0.01, the conversion into DMSO may decline. If the reaction is effected at a NOx/DMS ratio by weight of higher than 0.2, DMSO₂ may be produced, possibly in a large amount.

Furthermore, in a process embodying the present invention, it is especially preferable that the oxygen concentration of the gas mainly composed of oxygen (oxygen gas) used as an oxidizing agent is 80 wt% or more. If a gas with an oxygen concentration of lower than 80 wt% is used as an oxidizing agent, the conversion into DMSO tends to decline. To keep the conversion high, the reaction solution is retained for a period of time corresponding to more than twice that of an ordinary case.

As the oxygen gas, a highly pure oxygen gas with an oxygen concentration of 99 wt% or more can also be used, but in the present invention, a relatively low purity oxygen gas obtained by pressure swing adsorption treatment (hereinafter called "the PSA process") can also be used.

The PSA process refers to a method of enhancing the oxygen concentration in a gas by using the difference between oxygen and nitrogen in adsorbing power when the gas is adsorbed by an adsorbent with the rise of pressure and desorbed with the lowering of pressure. As the adsorbent, zeolite is mainly used. In this PSA process, the oxygen concentration in the gas obtained with air as the raw material can be freely adjusted in a range from about 21 wt% to 96 wt%. In a process embodying the present invention, an oxygen gas with an oxygen concentration of 80 wt% to 96 wt%, obtainable by treating air by the PSA process, can be used.

In the present invention, for efficient oxidation of DMS, it is preferable that the amount of the oxygen gas supplied to the oxidation reaction system is more than the theoretical amount, and the extra unreactive oxygen gas is released outside the reaction system as a reaction off gas together with NOx used as a catalyst. In this case, the NO concentration in the NOx in the reaction off gas is usually 5 wt% to 60 wt%, though depending on the process conditions.

The oxygen concentration in the reaction off gas is usually 10 wt% to 50 wt%, though also depending on the reaction conditions. It is preferable that the oxygen gas is supplied into the oxidation reactor by an amount slightly larger than the theoretical amount as described above, and it is preferable that the oxygen concentration in the reaction off gas is 20 wt% or more.

Moreover, in order to raise the NOx absorption efficiency in the present invention, it is preferable that NO in the reaction off gas is oxidized into NO₂ in a gas phase NO oxidizing reactor by oxygen in the mixed gas consisting of NOx and oxygen released as off gas from the liquid phase reactor.

Furthermore, in a process embodying the present invention, in order to raise the NO oxidation efficiency, it is preferable to insert a cooler between the liquid phase reactor and the NO oxidizing reactor. The temperature in the NO oxidizing reactor is preferably lower than 50°C, more preferably lower than 30°C. Although various types of reactor are available for the NO oxidation reaction, a tubular reactor is especially preferred. It is preferable that the pressure in the NO oxidation reactor during the oxidation reaction is atmospheric pressure, but the reaction can take place even at higher pressure. Preferably, the NO concentration in the NOx is less than 5wt.%.

Thus, as indicated above, it is preferable that the oxygen concentration in the reaction off gas is 20 wt% or more because, if the oxygen concentration in the reaction off gas is lower than 20 wt%, the abovementioned NO oxidation efficiency may decline.

In a process embodying the present invention, in the reaction product solution mainly composed of DMSO delivered from the oxidation reactor, the NOx which has not been released as the reaction off gas is dissolved by an amount corresponding to the solubility of NOx in DMSO. In such a process embodying the present invention, the dissolved NOx may be removed from the reaction product solution mainly composed of DMSO, by blowing nitrogen gas into the reaction product solution. In this case, the preferable temperature of the reaction product solution into which nitrogen gas is blown is 40°C to 90°C. A more preferable range is 50°C to 80°C. It is preferable that the amount of the nitrogen to be blown in is 1 wt% to 7 wt% based on the weight of the reaction product solution. A more preferable range is 2 wt% to 6 wt%.

Furthermore, in a process embodying the present invention, the reaction off gas and/or the gas removed from the reaction product solution respectively containing NOx is passed through a NOx absorbing column, using the reaction product solution remaining after removing NOx as a NOx absorbable solution, to recover NOx from the reaction off gas and/or the gas removed from the reaction product solution.

The absorption efficiency is not so greatly affected by the type of the NOx absorbing column, but a packed column, plate column or wetted wall column are preferably used.

It is preferable that the amount of the reaction product solution mainly composed of DMSO remaining after removing NOx used as the absorbing solution of the absorbing column is 5 times or more the amount of NOx. It is more preferable that the amount is 10 times or more.

### Examples

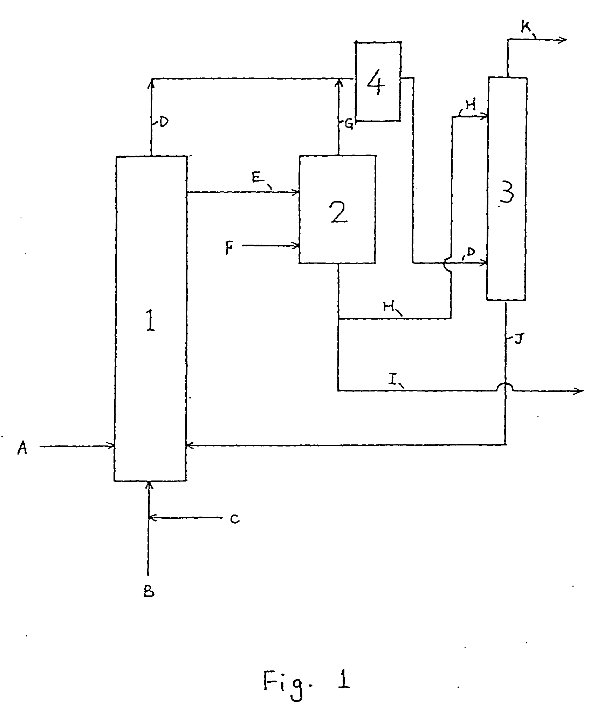

Preferred embodiments of the present invention are described below in still more detail with reference to the following Examples and accompanying drawing (Fig.1), which is a model diagram showing the outline of an apparatus for recovery of NOx which is useful in a process embodying the present invention.

### Example 1

As indicated above, Fig. 1 is a model diagram for illustrating the outline of the recovery equipment and process used for recovering NOx of the present invention.

With reference to Fig. 1, DMS was supplied from a pipe A at a rate of 100 g per hour (hereinafter expressed as (g/h)), catalyst NOx, from a pipe B at a rate of 0.6 (g/h), and oxygen, from a pipe C at a rate of 30.5 (g/h), respectively to an oxidation reactor 1 (4 cm dia. x 50 cm), and oxidation reaction was effected at a reactor temperature of 30°C. The reaction off gas containing oxygen and NOx was supplied through a pipe D to a NO oxidizing reactor 4 and thence to a NOx absorbing column 3 (1 cm dia. x 50 cm) for recovery of NOx. To a degassing tank 2 (2 cm dia. x 20 cm), the reaction product solution produced in the oxidation reactor 1 was supplied from a pipe E, and furthermore nitrogen gas was supplied from a pipe F at a rate of 10 (g/h), to remove NOx in the reaction product solution. The temperature of the degassing tank 2 was 50°C. The NOx removed from the reaction product solution was supplied into the NO oxidizing reactor 4 and thence to the NOx absorbing column 3 through pipes G and D. Furthermore, the reaction solution product mainly composed DMSO from a pipe H was supplied into the NOx absorbing column 3 as a NOx absorbable solution. The amount of the reaction product solution supplied in this case was 50.6 (g/h). The reaction product solution not supplied as the NOx absorbable solution to the NOx absorbing column 3 was supplied to the subsequent step from a pipe I.

As described above, to the NOx absorbing column 3, the NOx absorbable solution was supplied from the pipe H to an upper portion of the absorbing column, and the catalyst NOx was supplied from the pipe D to a lower portion of the absorbing column, to let the reaction product solution absorb NOx in the NOx absorbing column 3. The temperature in the NOx absorbing column 3 in this case was 20°C. The oxygen and nitrogen supplied to the NOx absorbing column 3 together with the catalyst NOx from the pipe D were released from the top of the NOx absorbing column 3 through a pipe K as an off gas. The amounts of oxygen and nitrogen released in this case were 12.2 (g/h) and 10 (g/h) respectively.

The NOx-containing solution released from the NOx absorbing column was recycled from a pipe J into the oxidation reactor 1. The amount of the NOx absorbed in this case was 51 (g/h), and the amount of NO₂ in the NOx-containing solution was 5.6 (g/h). As a result of the recycling of the NOx absorbable solution, continuous operation could be executed by supplying the catalyst NO₂ by 0.6 (g/h) which corresponded to about one tenth of the otherwise required amount.

### Example 2

With reference to Fig. 1, DMS was supplied from the pipe A at a rate of 100 (g/h), catalyst NOx, from the pipe B at a rate of 6.2 (g/h) and a gas with an oxygen concentration of 90% obtained by PSA, from the pipe C at a rate of 34.0 (g/h), respectively to the oxidation reactor 1, and oxidation reaction was effected at a reactor temperature of 30°C. The reaction product solution mainly composed of DMSO produced in the oxidation reactor 1 was supplied from the pipe E to the subsequent step. The amount of the reaction product solution supplied in this case was 124.8 (g/h). Furthermore, the reaction off gas containing oxygen and NOx was supplied from the pipe D to the subsequent step. The amounts of oxygen and NOx in the reaction off gas in this case were 12.2 (g/h) and 2.4 (g/h) respectively. The conversion into DMSO in this DMS liquid phase continuous oxidation was more than 99%.

### Example 3

With reference to Fig. 1, DMS was supplied from the pipe A at a rate of 100 (g/h), catalyst NOx, from the pipe B at a rate of 0.6 (g/h) and a gas with an oxygen concentration of 90% obtained by PSA, from the pipe C at a rate of 34.0 (g/h), respectively to the oxidation reactor 1, and oxidation reaction was effected at a reactor temperature of 30°C. The reaction off gas containing oxygen and NOx was supplied through the pipe D to the NO oxidizing reactor 4 and thence to the NOx absorbing column 3 for recovery of NOx. The reaction product solution produced in the oxidation reactor 1 was supplied from the pipe E to the degassing tank 2 at a rate of 124.5 (g/h), and furthermore nitrogen gas was supplied to the degassing tank 2 from the pipe F at a rate of 10 (g/h), to remove NOx in the reaction product solution. The temperature of the degassing tank 2 in this case was 50°C. The removed NOx was supplied to the NO oxidizing reactor 4 and thence to the NOx absorbing column 3 through the pipes G and D. Furthermore, the reaction product solution mainly composed of DMSO was supplied from the pipe H to the NOx absorbing column 3 as a NOx absorbable solution. The amount of the reaction product solution supplied in this case was 50.9 (g/h). The reaction product solution which was not supplied to the NOx absorbing column as the NOx absorbable solution was supplied to the subsequent step from the pipe I.

As described above, to the NOx absorbing column 3, the NOx absorbable solution from the pipe H was supplied to an upper portion of the absorbing column and the catalyst NOx was supplied to a lower portion of the absorbing column from the pipe D, to let the reaction product solution absorb NOx. The temperature in the NOx absorbing column in this case was 20°C. The oxygen and nitrogen supplied with the catalyst NOx to the NOx absorbing column 3 from the pipe D were released as the off gas from the top of the absorbing column through the pipe K. The amounts of oxygen and nitrogen released in this case were 11.9 (g/h) and 10 (g/h) respectively.

The NOx-containing solution released from the NOx absorbing column was recycled into the oxidation reactor 1 through the pipe J. The amount of the NOx-containing solution in this case was 51 (g/h), and the amount of NO₂ in the NOx-containing solution was 5.6 (g/h). As a result of the recycling of the NOx-containing solution, continuous operation could be effected by supplying the catalyst NOx by 0.6 (g/h) which corresponded to about one tenth of the otherwise required amount. The conversion into DMSO in this case was 99%.

### [Effects of the invention]

Since, in accordance with processes embodying the present invention, catalyst NOx is recycled for use, the amount of the alkali used as a treating solution can be decreased, and industrial waste treatment is unnecessary. Furthermore, since no NOx leaks into the atmosphere, the resource can be effectively used and an environmental problem can be solved, as compared with the conventional process. Furthermore, the intended DMSO can be produced at a low cost.

That is, in a process embodying the present invention, during the continuous oxidation reaction of DMS for producing DMSO, the NOx used as a catalyst is recycled for use, to improve the conversion from DMS into DMSO, thus allowing efficient production of DMSO. More specifically, the NOx recovered from the reaction off gas and/or the gas removed from the reaction product solution is re-used, to improve the recovery rate and/or absorption rate of NOx.

Thus, processes embodying the present invention allow industrial advantageous production of DMSO which is widely used as a reaction solvent of medical and agricultural intermediate products, a synthetic reagent, a special detergent for electronic material, etc. or as a solvent of resins, films and fibres.

Furthermore, processes embodying the present invention allow the production of DMSO at a low cost using simplified equipment, by using a relatively low pure oxygen gas obtained by the pressure swing adsorption treatment of air as an oxidizing agent.

## Claims

1. A process for producing dimethyl sulfoxide, in which dimethyl sulfide is continuously oxidized by a gas mainly composed of oxygen using NOx as a catalyst in a liquid phase, comprising the step of supplying at least partially the dimethyl sulfoxide and NOx of the reaction product solution, obtained by the said oxidation reaction, as a reaction solution to the oxidation reaction for recycling.

2. A process according to claim 1, in which the dimethyl sulfide is continuously oxidized in a liquid phase using an oxidation reactor, the process further comprising the steps of allowing the dimethyl sulfoxide solution to absorb NOx present in reaction off gas in an absorbing column, and supplying the NOx-containing dimethyl sulfoxide solution to the oxidation reactor for recycling.

3. A process according to claim 1 or 2, wherein nitrogen gas is brought into contact with the reaction product solution at a temperature higher than the oxidation reaction temperature, to remove NOx from the reaction product solution mainly composed of dimethyl sulfoxide.

4. A process according to claim 3, wherein NOx gas removed from the reaction product solution is sent to a NOx absorbing column, to allow dimethyl sulfoxide solution to absorb NOx.

5. A process according to claim 3 or 4, wherein the reaction product solution remaining after removal of the NOx therefrom is used as a NOx absorbable solution.

6. A process according to any one of claims 2 to 5, wherein NO in the reaction off gas and/or in the gas removed from the reaction product solution is oxidized, in an NO oxidizing reactor, into NO₂, and then the NOx is absorbed.

7. A process according to any one of Claims 1 to 6, comprising the step of using oxygen gas obtained by the pressure swing adsorption treatment of air, as the gas mainly composed of oxygen.

8. A process according to any preceding claim wherein the oxygen concentration of the gas mainly composed of oxygen is 80wt% or more.

## Patentansprüche

1. Verfahren zur Herstellung von Dimethylsulfoxid, worin Dimethylsulfid mit einem hauptsächlich aus Sauerstoff bestehenden Gas unter Verwendung von NOx als Katalysator in einer flüssigen Phase kontinuierlich oxidiert wird, umfassend den Schritt der zumindest teilweisen Zufuhr des Dimethylsulfoxids und des NOx der durch die Oxidationsreaktion erhaltenen Reaktionsproduktlösung als Reaktionslösung zur Oxidationsreaktion zur Rezyklierung.

2. Verfahren nach Anspruch 1, worin das Dimethylsulfid in einer flüssigen Phase unter Verwendung eines Oxidationsreaktors kontinuierlich oxidiert wird, wobei das Verfahren weiters folgende Schritte umfasst: Absorption des im Reaktionsabgas enthaltenen NOx durch die Dimethylsulfoxidlösung in einer Absorptionskolonne und Zufuhr der NOx-hältigen Dimethylsulfoxidlösung zum Oxidationsreaktor zur Rezyklierung.

3. Verfahren nach Anspruch 1 oder 2, worin Stickstoffgas bei einer Temperatur über der Oxidationsreaktionstemperatur mit der Reaktionsproduktlösung in Kontakt gebracht wird, um NOx aus der Reaktionsproduktlösung, die hauptsächlich aus Dimethylsulfoxid besteht, zu entfernen.

4. Verfahren nach Anspruch 3, worin das aus der Reaktionsproduktlösung entfernte NOx-Gas einer NOx-Absorptionskolonne zugeführt wird, um die Absorption von NOx durch die Dimethylsulfoxidlösung zu ermöglichen.

5. Verfahren nach Anspruch 3 oder 4, worin die nach Entfernen des NOx daraus zurückbleibende Reaktionsproduktlösung als NOx-absorbierfähige Lösung eingesetzt wird.

6. Verfahren nach einem der Ansprüche 2 bis 5, worin NO im Reaktionsabgas und/oder in dem aus der Reaktionsproduktlösung entfernten Gas in einem NO-Oxidationsreaktor zu NO₂ oxidiert wird und anschließend NOx absorbiert wird.

7. Verfahren nach einem der Ansprüche 1 bis 6, umfassend den Schritt der Verwendung von Sauerstoffgas, das durch eine Behandlung von Luft mittels Druckänderungsadsorption erhalten wird, als das hauptsächlich aus Sauerstoff bestehende Gas.

8. Verfahren nach einem der vorangegangenen Ansprüche, worin die Sauerstoffkonzentration des hauptsächlich aus Sauerstoff bestehenden Gases 80 Gew.-% oder mehr beträgt.

## Revendications

1. Procédé de production de diméthyl sulfoxyde, dans lequel du diméthyl sulfure est continuellement oxydé par un gaz composé principalement d'oxygène en utilisant NOx comme catalyseur dans une phase liquide, comprenant l'étape de fournir au moins partiellement le diméthyl sulfoxyde et NOx de la solution de produit réactionnel, obtenue par ladite réaction d'oxydation en tant que solution de réaction, à la réaction d'oxydation pour le recyclage.

2. Procédé selon la revendication 1, dans lequel le diméthyl sulfure est continuellement oxydé dans une phase liquide en utilisant un réacteur d'oxydation, le procédé comprenant de plus les étapes de permettre à la solution du diméthyl sulfoxyde d'absorber NOx présent dans le gaz d'échappement de la réaction dans une colonne absorbante et de fournir la solution du diméthyl sulfoxyde contenant NOx au réacteur d'oxydation pour le recyclage.

3. Procédé selon la revendication 1 ou 2, où de l'azote gazeux est mis en contact avec la solution du produit réactionnel à une température supérieure à la température de la réaction d'oxydation pour éliminer NOx de la solution du produit réactionnel principalement composée de diméthyl sulfoxyde.

4. Procédé selon la revendication 3, où le gaz NOx éliminé de la solution du produit réactionnel est envoyé à une colonne absorbant NOx pour permettre à la solution du diméthyl sulfoxyde d'abosber NOx.

5. Procédé selon la revendication 3 ou 4, où la solution du produit réactionnel restant après élimination de NOx est utilisée en tant que solution absorbable de NOx.

6. Procédé selon l'une quelconque des revendications 2 à 5, où NO dans les gaz d'échappement de la réaction et/ou dans le gaz retiré de la solution du produit réactionnel est oxydé, dans un réacteur d'oxydation de NO, en NO₂, et puis NOx est absorbé.

7. Procédé selon l'une quelconque des revendications 1 à 6, comprenant l'étape d'utiliser l'oxygène gazeux obtenu par le traitement par adsorption avec oscillation de pressions de l'air, en tant que le gaz principalement composé d'oxygène.

8. Procédé selon toute revendication précédente où la concentration en oxygène du gaz principalement composé d'oxygène est de 80% en poids ou plus.
